# EUROPEAN PATENT APPLICATION

(11) **EP 4 806 990 A1**
(43) Date of publication of application: **16.09.2026**
(21) Application number: 24887750.8
(22) Date of filing: 23.10.2024
(51) Int. Cl.: C12N 5/10, C07K 16/00, C12N 15/90, C12N 9/22

(54) **CELL STRAIN, AND PREPARATION METHOD THEREFOR AND USE THEREOF**

(30) Priority: 08.11.2023 CN 202311484805
(71) Applicant: Shenzhen Taili Biotechnology Co., Ltd, Shenzhen, Guangdong 518017 (CN)
(72) Inventor: CHEN, Liang, Shenzhen, Guangdong 518017 (CN); ZHANG, Ning, Shenzhen, Guangdong 518017 (CN); LIN, Longzhi, Shenzhen, Guangdong 518017 (CN); LIANG, Guolong, Shenzhen, Guangdong 518017 (CN)
(74) Representative: Valet Patent Services Limited
(86) International application number: PCT/CN2024/126623
(87) International publication number: WO 2025/098137

(57) **Abstract**

The present invention relates to the technical field of biology, and in particular, provided are a cell strain and a preparation method therefor and the use thereof. The cell strain contains a highly expressed fragment and an exogenous nucleic acid fragment integrated into the highly expressed fragment. The highly expressed fragment contains a nucleotide sequence as shown in SEQ ID NO. 1. The exogenous nucleic acid fragment contains a first target gene encoding a first protein fragment and a second target gene encoding a second protein fragment; the expression difficulty of the first protein fragment is higher than that of the second protein fragment; and the first target gene is located upstream of the second target gene. By means of the highly expressed fragment, stable and high expression of target proteins can be realized. In addition, by means of limiting the sequence of the target proteins, the purposes of the high expression of the target proteins, the balanced expression of various target proteins and the reduction in the mismatch rate of the target proteins can be achieved.

## Description

### CROSS REFERENCE TO RELATED DISCLOSURES

The present application claims the priority to the Chinese patent application with filing No. 2023114848053, entitled "CELL STRAIN, AND PREPARATION METHOD THEREFOR AND USE THEREOF", and filed on November 8, 2023 with the Chinese Patent Office, the contents of which are incorporated herein by reference in their entirety.

### TECHNICAL FIELD

The present application relates to the field of biotechnology, and specifically provides a cell strain, a preparation method therefor, and a use thereof.

### BACKGROUND ART

Chinese hamster ovary (CHO) cells are the expression host for the commercial production of recombinant proteins. Currently, various combinations of biological therapeutic drugs produced from CHO cells have been approved for marketing, including multiple monoclonal antibodies (mAbs) and other proteins. Since the emergence of production processes for CHO cell cultures more than 30 years ago, those skilled in the art have made unremitting efforts to significantly improve the production capacity of CHO cells. Particularly, the titers of past industrial antibody production were typically far below 1 g/L. However, the antibody production of manufacture teams has now achieved titers of g/L or above, typically at levels of 3 g/L or above. However, currently, in the upstream cell strain construction and screening stage, non-targeted transgene integration methods are still habitually used to generate a stably transfected CHO cell library, and the cell lines are established and produced from the CHO cell library. Therefore, it is necessary to identify those clones with suitable production characteristics through tedious screening from a highly heterogeneous cell library. Since these screened cell strains are often multi-copy, the stabilities of different sites in the cell strains vary greatly. In contrast, site-specific integration provides means to generate more consistent clones, and this technique reduces the cell line development timeline. Therefore, site-specific integration has become a promising strategy, by which CHO cell lines may repeatedly target their preferred genomic sites that are highly active and support stable expression. However, in practical uses of site-specific integration, the expression results still require improvement, and the problem of high mismatch rate still exists.

### SUMMARY

The first purpose of the present application is to provide a cell strain.

The second purpose of the present application is to provide a method for preparing the aforementioned cell strain.

The third purpose of the present application is to provide a use of the aforementioned cell strain.

To achieve the aforementioned purposes, the present application adopts the following technical solutions.

A cell strain, where the cell strain includes a high-expression fragment and an exogenous nucleic acid fragment integrated within the high-expression fragment;
the high-expression fragment contains the nucleotide sequence as shown in SEQ ID No: 1; and
the exogenous nucleic acid fragment includes a first target gene encoding a first protein fragment and a second target gene encoding a second protein fragment; the expression difficulty of the first protein fragment is higher than that of the second protein fragment, and the first target gene is located upstream of the second target gene.

Further, the exogenous nucleic acid fragment further includes a third target gene encoding a third protein fragment and a fourth target gene encoding a fourth protein fragment, the expression difficulty of the third protein fragment is higher than that of the fourth protein fragment, and the third target gene is located upstream of the fourth target gene, where the first target gene, the second target gene, the third target gene, and the fourth target gene jointly encode a bispecific antibody or an antigen-binding fragment thereof.

Further, the second target gene is located upstream of the third target gene.

Further, the first protein fragment is a first light chain, the second protein fragment is a second light chain, the third protein fragment is a first heavy chain, and the fourth protein fragment is a second heavy chain; optionally, the protein formed by binding of the first light chain and the first heavy chain may specifically bind to a first target, and the protein formed by binding of the second light chain and the second heavy chain may specifically bind to a second target.

Further, the sequence of the first target gene is as shown in SEQ ID No: 2, the sequence of the third target gene is as shown in SEQ ID No: 3, the sequence of the second target gene is as shown in SEQ ID No: 4, and the sequence of the fourth target gene is as shown in SEQ ID No: 5.

Further, each of the first target gene and the second target gene contains upstream a nucleotide sequence encoding the first signal peptide as shown in SEQ ID No: 6, and each of the third target gene and the fourth target gene contains upstream a nucleotide sequence encoding the second signal peptide as shown in SEQ ID No: 7.

Further, the integration site of the exogenous nucleic acid fragment is any site within the base interval from positions 11 to 430 of the high-expression fragment;
optionally, the integration site of the exogenous nucleic acid fragment is any site within the base interval from positions 21 to 414 of the high-expression fragment;
optionally, the integration site of the exogenous nucleic acid fragment is any site within the base interval from positions 38 to 402 of the high-expression fragment;
optionally, the integration site of the exogenous nucleic acid fragment is any site within the base interval from positions 53 to 389 of the high-expression fragment;
optionally, the integration site of the exogenous nucleic acid fragment is any site within the base interval from positions 73 to 373 of the high-expression fragment;
optionally, the integration site of the exogenous nucleic acid fragment is any site within the base interval from positions 91 to 360 of the high-expression fragment;
optionally, the integration site of the exogenous nucleic acid fragment is any site within the base interval from positions 108 to 342 of the high-expression fragment;
optionally, the integration site of the exogenous nucleic acid fragment is any site within the base interval from positions 126 to 326 of the high-expression fragment;
optionally, the integration site of the exogenous nucleic acid fragment is any site within the base interval from positions 143 to 310 of the high-expression fragment;
optionally, the integration site of the exogenous nucleic acid fragment is any site within the base interval from positions 160 to 295 of the high-expression fragment;
optionally, the integration site of the exogenous nucleic acid fragment is any site within the base interval from positions 178 to 274 of the high-expression fragment;
optionally, the integration site of the exogenous nucleic acid fragment is any site within the base interval from positions 194 to 263 of the high-expression fragment;
optionally, the integration site of the exogenous nucleic acid fragment is any site within the base interval from positions 209 to 253 of the high-expression fragment;
optionally, the integration site of the exogenous nucleic acid fragment is any site within the base interval from positions 221 to 242 of the high-expression fragment;
optionally, the integration site of the exogenous nucleic acid fragment is any site within the base interval from positions 231 to 240 of the high-expression fragment; and
optionally, the integration site of the exogenous nucleic acid fragment is the position indicated by the annotation information NW_003616785.1:83044 in CHO cells.

Further, the cell strain is a CHO cell.

A preparation method for the cell strain, where the preparation method includes the following steps: site-specifically integrating the exogenous nucleic acid fragment into the high-expression fragment to obtain the cell strain.

Further, the preparation method includes the following steps:
S1: providing the exogenous nucleic acid fragment, where the exogenous nucleic acid fragment includes, in the 5'-3' direction, a first target gene encoding a first light chain, a second target gene encoding a second light chain, a third target gene encoding a first heavy chain, and a fourth target gene encoding a second heavy chain in sequence, the expression difficulty of the first target gene is higher than that of the second target gene, and the expression difficulty of the third target gene is higher than that of the fourth target gene; and the first target gene, the second target gene, the third target gene, and the fourth target gene are each independently provided upstream with a nucleotide sequence encoding a signal peptide;
the first target gene, the second target gene, the third target gene, and the fourth target gene jointly encode a bispecific antibody or an antigen-binding fragment thereof; and
S2. site-specifically integrating the exogenous nucleic acid fragment into the high-expression fragment to obtain the cell strain.

Use of the aforementioned cell strain in protein expression.

Compared with the prior art, the technical effects of the present application are as follows.

The present application screens and analyzes gene sites within a CHO cell to obtain insertion sites and high-expression fragments suitable for site-specific recombination (or site-specific integration) technique. It is found through further researches that in the case of multiple target proteins, when the gene encoding a protein that is relatively difficult to express is located upstream of the gene encoding a protein that is relatively easy to express, i.e., by defining the gene order of the target protein, it is possible to achieve the purpose of high expression of the target protein, balanced expression of multiple target proteins, and reduced mismatch rate of the target protein.

### BRIEF DESCRIPTION OF DRAWINGS

To more clearly illustrate the technical solutions in the embodiments of the present application or the prior art, the drawings required to be used in the description of the embodiments or the prior art will be briefly introduced below. Obviously, the drawings described below are some embodiments of the present application. For those ordinarily skilled in the art, other drawings may be obtained according to the provided drawings without paying creative effort.
FIG. 1 is an operational flowchart view of the high-expression fragment screening in Example 1;
FIG. 2 is a structural schematic view of the selection site plasmid in step (1) of Example 1;
FIG. 3 is a structural schematic view of the pDonor0.0-H plasmid in step (1) of Example 2;
FIG. 4 is a structural schematic view of the pDonor0.0-L plasmid in step (1) of Example 2;
FIG. 5 is a structural schematic view of the pLanding0.0 plasmid in step (1) of Example 2;
FIG. 6 is a structural schematic view of pLanding0.0-AL-VL-AH-VH plasmid in step (1) of Example 2;
FIG. 7 is a structural schematic view of pLanding0.0-AL-VL-VH-AH plasmid in step (1) of Example 2;
FIG. 8 is a structural schematic view of pLanding0.0-VL-AL-VH-AH plasmid in step (1) of Example 2;
FIG. 9 is a structural schematic view of pLanding0.0-VL-AL-AH-VH plasmid in step (1) of Example 2;
FIG. 10 is a map of the pGBB Bxb-1 integrase plasmid in step (2) of Example 2; and
FIG. 11 is a view of the SDS-PAGE analysis results in step (6) of Example 2.

### DETAILED DESCRIPTION OF EMBODIMENTS

### Definition

In the present application, "further", "more further", and "particularly", etc. are used for descriptive purposes to indicate difference in content, but should not be construed as limiting the scope of protection of the present application.

In the present application, "optionally", "optional", and "selectable" mean that they are optional, that is, they are selected from any one of two parallel technical solutions of "present" and "absent". If "optional" occurs in multiple places in a technical solution, unless otherwise specified, without contradictions or mutual constraints, each "optional" is independent.

"Multiple", "various", "multiple times", "multivariate", etc., involved in the present application, unless otherwise specified, refer to a quantity greater than 2 or equal to 2. For example, "one or more" means one or more than or equal to two.

The phrase "high-expression fragment" refers to a nucleotide sequence in the cell genome, which nucleotide sequence, when being added (i.e., integrated) with a suitable gene or construct exogenously, exhibits a higher level of expression, particularly the high-level expression at the protein level, compared with other regions or sequences in the genome, particularly referring to high-level expression at the protein level.

The phrase "exogenous nucleic acid fragment" refers to any DNA sequence or gene that is not present in a high-expression fragment found in nature. For example, the "exogenous nucleic acid fragment" in a high-expression fragment (e.g., a high-expression fragment containing the sequence SEQ ID No: 1) of CHO may be a hamster gene not found in a high-expression fragment of a specific CHO in nature (i.e., the hamster gene is derived from another nucleotide sequence of the hamster genome), a gene from any other species (e.g., a human gene), a chimeric gene (e.g., human/mouse), or any other gene not found in nature and present in the target CHO high-expression fragment.

The phrase "high expression difficulty" refers to proteins that are difficult to produce. For example, in the same expression environment, the production of proteins with "high expression difficulty" during transcription, translation, folding, or other processes is more difficult than that of other proteins. For example, in the embodiments of the present application, the constant region CL in the ANG-2 antibody light chain sequence and the constant region CH1 in the ANG-2 antibody heavy chain sequence are mutually exchanged, and since artificial sequence exchange is more likely to cause mismatches, the expression difficulty of the ANG-2 antibody light chain sequence is higher than that of the VEGF antibody light chain sequence, and the expression difficulty of the ANG-2 antibody heavy chain sequence is higher than that of the VEGF antibody heavy chain sequence.

The phrase "antigen-binding fragment" includes a protein having at least one complementarity-determining region (CDR) and capable of selectively recognizing an antigen, i.e., capable of binding to an antigen with an equilibrium dissociation constant (KD) at least in the range of micromolar.

The phrase "bispecific antibody" (bsAb) includes an antibody that may selectively bind to two epitopes. Bispecific antibodies generally include two different heavy chains, of which each heavy chain specifically binds to a different epitope-either on two different molecules (e.g., antigens) or on the same molecule (e.g., on the same antigen).

Bispecific antibodies exist in a variety of forms, for example, Amgen has evolved from the BiTE platform, a small protein consisting of only two tandem antigen-binding fragments, to IgG-like immunoglobulins with additional structure domains, for example, the marketed cadonilimab from Akeso Biopharma. Currently, over 20 different commercial technique platforms may be available for the creation and development of bispecific antibodies, over ten bispecific antibodies are launched on the market, and over 100 bispecific antibodies are in clinical development. However, a pain point in the industry is how to screen for a bispecific antibody-expressing cell strain that meets the expected quality and is suitable for the later-stage manufacturing process. Due to the unique structural characteristics of bispecific antibodies, the structures designed by different platforms are vastly different. Therefore, the manufacturing process of bispecific antibodies presents significant challenges, and an excellent cell strain, as the starting point of the entire manufacturing process, determines, to a large extent, the complexity degree of the later-stage process and the manufacturing cost after the product is launched on the market in the later-stage, making the screening for an excellent cell strain particularly important for the entire pharmaceutical enterprise.

The phrase "site-specific integration" (also known as "site-specific recombination" or "site-special recombination") refers to a gene-targeting method used to guide the insertion or integration of a gene or nucleic acid sequence at a specific position in the genome, that is, to guide DNA to a specific site between two nucleotides in a linked polynucleotide chain.

The applicant discovers through researches a CHO nucleotide sequence capable of stably and highly expressing the target protein, which serves as a high-expression fragment for the target protein. Further, through site-specific integration techniques, it is found that inserting (or integrating) the nucleotide sequence encoding the target protein as an exogenous nucleic acid fragment into the high-expression fragment may greatly reduce the construction cost of cell strain while simultaneously achieving stable and high expression of the target protein. More further researches reveal that in the site-specific integration technique, when multiple nucleotide sequences are involved within the nucleotide sequence encoding the target protein, the arrangement order of the multiple nucleotide sequences may affect the expression level of the respective encoded proteins thereof and also has an impact on the mismatch rate of the target protein, thereby giving rise to the present application.

The present application discloses a cell strain, where the cell strain includes a high-expression fragment and an exogenous nucleic acid fragment integrated within the high-expression fragment; the high-expression fragment includes the nucleotide sequence as shown in SEQ ID No: 1; and the exogenous nucleic acid fragment includes a first target gene encoding a first protein fragment and a second target gene encoding a second protein fragment, the expression difficulty of the first protein fragment is higher than that of the second protein fragment, and the first target gene is located upstream of the second target gene.

In the present application, the integration site of the exogenous nucleic acid fragment may correspond to the position 1, 2, 3, 4, 5, 6, 7, 8, 9, 10, ... 444 of the high-expression fragment.

The aforementioned "cell strain" includes any cell suitable for expressing a recombinant nucleic acid sequence and possessing a high-expression fragment that allows for stable integration and enhanced expression of exogenous nucleic acid fragments. The cell includes a mammalian cell, such as non-human animal cell, human cell, or cell fusion product, such as hybridoma or quadroma. In some embodiments, the cell is human, monkey, ape, hamster, rat, or mouse cell. In some embodiments, the cell is a mammalian cell selected from the following cells: CHO (e.g., CHO K1, etc.), COS (e.g., COS-7), retinal cell, Vero, CV1, kidney (e.g., HEK293, 293EBNA, MDCK, etc.), HeLa, HepG2, myeloma cell, and tumor cell. Specifically, the cell strain contains a high-expression fragment of the nucleotide sequence as shown in SEQ ID No: 1; and on this basis, by integrating an exogenous nucleic acid fragment into the high-expression fragment, a cell strain stably and highly expressing the target protein may be obtained. Preferably, the cell strain is the CHO cell.

The exogenous nucleic acid fragment encodes the target protein; and when the target protein is composed of multiple parts (for example, a single-chain antibody is mainly composed of a heavy chain variable region and a light chain variable region), the coding sequence of a protein with relatively high expression difficulty is preferably arranged upstream of the coding sequence of a protein with relatively low expression difficulty, where this arrangement is conducive to balancing the expression levels of the multiple parts, improving the expression level of the target protein and reducing the mismatch rate.

In some embodiments, the exogenous nucleic acid fragment includes a first target gene encoding a first protein fragment, a second target gene encoding a second protein fragment, a third target gene encoding a third protein fragment, and a fourth target gene encoding a fourth protein fragment. The expression difficulty of the first protein fragment is higher than that of the second protein fragment, and the first target gene is located upstream of the second target gene; and the expression difficulty of the third protein fragment is higher than that of the fourth protein fragment, and the third target gene is located upstream of the fourth target gene. Meanwhile, the first target gene, the second target gene, the third target gene, and the fourth target gene jointly encode a bispecific antibody or the antigen-binding fragment thereof.

Based on the above, to facilitate balancing the expression levels of the multiple parts, improving the expression level of the target protein and reducing the mismatch rate, the coding sequence of a protein with relatively high expression difficulty is preferably arranged upstream of the coding sequence of a protein with relatively low expression difficulty.

When the target protein is a bispecific antibody or an antigen-binding fragment thereof, the expression of the heavy chain depends on the correct folding of the light chain, and the light chain may assist in the secretion of the heavy chain, therefore the sequence encoding the light chain is preferably arranged upstream to facilitate the generation of more light chain antibody fragments and thus enable further increase of the yield of the target protein. Further, within the light chain part or heavy chain part, the coding sequence of a protein with relatively high expression difficulty is preferably arranged upstream of the coding sequence of a protein with relatively low expression difficulty, thereby further increasing the expression level of the bispecific antibody or the antigen-binding fragment thereof, and reducing the mismatch rate.

In some embodiments, further, the second target gene is located upstream of the third target gene.

In specific embodiments, the structure of the bispecific antibody or the antigen-binding fragment thereof is as follows: the first protein fragment is a first light chain, the second protein fragment is a second light chain, the third protein fragment is a first heavy chain, and the fourth protein fragment is a second heavy chain. In some embodiments, the protein formed by binding of the first light chain and the first heavy chain may specifically bind to a first target; and the protein formed by binding of the second light chain and the second heavy chain may specifically bind to a second target.

In more specific embodiments, the sequence of the first target gene is as shown in SEQ ID No: 2, the sequence of the third target gene is as shown in SEQ ID No: 3, the sequence of the second target gene is as shown in SEQ ID No: 4, and the sequence of the fourth target gene is as shown in SEQ ID No: 5. The target protein here is an ANG-2/VEGF bispecific bivalent antibody, where the antibody contains the heavy chain sequence (coding sequence SEQ ID No: 3) and the light chain sequence (coding sequence SEQ ID No: 2) corresponding to the ANG-2 target, and the heavy chain sequence (coding sequence SEQ ID No: 5) and the light chain sequence (coding sequence SEQ ID No: 4) corresponding to the VEGF target. In the above, the constant region CL of the ANG-2 antibody light chain sequence and the constant region CH1 within the ANG-2 antibody heavy chain sequence are mutually exchanged. Therefore, the expression difficulty of the ANG-2 antibody light chain sequence (coding sequence SEQ ID No: 2) is higher than that of the VEGF antibody light chain sequence (coding sequence SEQ ID No: 4), and the expression difficulty of the ANG-2 antibody heavy chain sequence (coding sequence SEQ ID No: 3) is higher than that of the VEGF antibody heavy chain sequence (coding sequence SEQ ID No: 5). Therefore, the order of the sequences integrated into the high-expression fragment is as follows: the first light chain gene (SEQ ID No: 2) which is difficult to express, the second light chain gene (SEQ ID No: 4) which is easy to express, the first heavy chain gene (SEQ ID No: 3) which is difficult to express, and the second heavy chain gene (SEQ ID No: 5) which is easy to express.

In some embodiments, the first target gene and the second target gene each contain upstream a nucleotide sequence encoding the first signal peptide as shown in SEQ ID No: 6, and the third target gene and the fourth target gene each contain upstream a nucleotide sequence encoding the second signal peptide as shown in SEQ ID No: 7.

In preferred embodiments, the integration site of the exogenous nucleic acid fragment is any site within the base interval from positions 11 to 430 of the high-expression fragment. In some embodiments, the integration site of the exogenous nucleic acid fragment is any site within the base interval from positions 21 to 414 of the high-expression fragment. In some embodiments, the integration site of the exogenous nucleic acid fragment is any site within the base interval from positions 38 to 402 of the high-expression fragment. In some embodiments, the integration site of the exogenous nucleic acid fragment is any site within the base interval from positions 53 to 389 of the high-expression fragment. In some embodiments, the integration site of the exogenous nucleic acid fragment is any site within the base interval from positions 73 to 373 of the high-expression fragment. In some embodiments, the integration site of the exogenous nucleic acid fragment is any site within the base interval from positions 91 to 360 of the high-expression fragment. In some embodiments, the integration site of the exogenous nucleic acid fragment is any site within the base interval from positions 108 to 342 of the high-expression fragment. In some embodiments, the integration site of the exogenous nucleic acid fragment is any site within the base interval from positions 126 to 326 of the high-expression fragment. In some embodiments, the integration site of the exogenous nucleic acid fragment is any site within the base interval from positions 143 to 310 of the high-expression fragment. In some embodiments, the integration site of the exogenous nucleic acid fragment is any site within the base interval from positions 160 to 295 of the high-expression fragment. In some embodiments, the integration site of the exogenous nucleic acid fragment is any site within the base interval from positions 178 to 274 of the high-expression fragment. In some embodiments, the integration site of the exogenous nucleic acid fragment is any site within the base interval from positions 194 to 263 of the high-expression fragment. In some embodiments, the integration site of the exogenous nucleic acid fragment is any site within the base interval from positions 209 to 253 of the high-expression fragment. In some embodiments, the integration site of the exogenous nucleic acid fragment is any site within the base interval from positions 221 to 242 of the high-expression fragment. In some embodiments, the integration site of the exogenous nucleic acid fragment is any site within the base interval from positions 231 to 240 of the high-expression fragment. In some implementations, the integration site of the exogenous nucleic acid fragment is the position indicated by the annotation information NW_003616785.1:83044 in the CHO cell.

The present application further discloses a method for preparing the cell strain, where when the cell is a CHO cell, the genome thereof contains the high-expression fragment of the present application, and the exogenous nucleic acid fragment is site-specifically integrated into the high-expression fragment to obtain the cell strain.

When the cell is another cell line, the genome thereof does not contain the high-expression fragment of the present application, and the high-expression fragment may be stably introduced into the cell first, and then the exogenous nucleic acid fragment is site-specifically integrated into the high-expression fragment to obtain the cell strain.

In some embodiments, the preparation method includes the following steps:
S1. providing an exogenous nucleic acid fragment, where the exogenous nucleic acid fragment includes a first target gene and a second target gene; and the expression difficulty of the first target gene is higher than that of the second target gene, and the first target gene is located upstream of the second target gene; and
S2. site-specifically integrating the exogenous nucleic acid fragment into the high-expression fragment to obtain the cell strain.

In some embodiments, the preparation method includes the following steps:
S1. providing an exogenous nucleic acid fragment, where the exogenous nucleic acid fragment includes a first target gene, a second target gene, a third target gene, and a fourth target gene; the expression difficulty of the first target gene is higher than that of the second target gene, and the first target gene is located upstream of the second target gene; the expression difficulty of the third protein fragment is higher than that of the fourth protein fragment, and the third target gene is located upstream of the fourth target gene; and
S2. site-specifically integrating the exogenous nucleic acid fragment into the high-expression fragment to obtain the cell strain.

In some embodiments, the preparation method includes the following steps:
S1: providing an exogenous nucleic acid fragment, where the exogenous nucleic acid fragment includes, in the 5'-3' direction, a first target gene encoding the first light chain, a second target gene encoding the second light chain, a third target gene encoding the first heavy chain, and a fourth target gene encoding the second heavy chain in sequence; the expression difficulty of the first target gene is higher than that of the second target gene, and the expression difficulty of the third target gene is higher than that of the fourth target gene; and the first target gene, the second target gene, the third target gene, and the fourth target gene are each independently provided upstream with a nucleotide sequence encoding a signal peptide;
the first target gene, the second target gene, the third target gene, and the fourth target gene jointly encode a bispecific antibody or an antigen-binding fragment thereof; and
S2. integrating the exogenous nucleic acid fragment into the high-expression fragment at a specific site to obtain the cell strain.

In specific embodiments, the preparation method includes the following steps:
S1: providing an exogenous nucleic acid fragment, where the exogenous nucleic acid fragment includes, in the 5'-3' direction, the nucleotide sequence encoding SEQ ID No: 6, SEQ ID No: 2, the nucleotide sequence encoding SEQ ID No: 6, SEQ ID No: 4, the nucleotide sequence encoding SEQ ID No: 7, SEQ ID No: 3, the nucleotide sequence encoding SEQ ID No: 7, and SEQ ID No. 5 in sequence; and
S2. site-specifically integrating the exogenous nucleic acid fragment into the high-expression fragment to obtain the cell strain.

The present application further discloses the use of the aforementioned cell strain in protein expression. In some embodiments, the protein includes an antigen binding moiety. In some embodiments, the antigen binding moiety is an antibody. In some embodiments, the antigen binding moiety is a bispecific antibody. In some embodiments, the bispecific antibody is a divalent antibody.

The present application will be further illustrated below through examples. Unless otherwise specified, the materials in the examples were prepared according to prior methods or purchased directly from the market.

### Example 1: Screening of High-expression Fragment

Specific consumables: Neon Resuspension Buffer R (ThermoFisher), which was a resuspension buffer dedicated for cell electroporation instrument; E1 Buffer (ThermoFisher), which was an electroporation buffer; a recovery medium, which was prepared by adding 1% GlutaMAX (ThermoFisher) to 80%(v/v) EX-CELL CHO Cloning Medium (Sigma-Aldrich) and 20%(v/v) EX-CELL Advanced CHO Fed-batch Medium (Sigma-Aldrich); an expansion medium and a subculture medium, which were both prepared by adding 1% GlutaMAX (ThermoFisher) to EX-CELL Advanced CHO Fed-batch Medium; a selective pressure medium 1, which was prepared by adding G418 to the subculture medium to a final concentration of 800 µg/ml; a selective pressure medium 2, which was prepared by adding Hygromycin to the subculture medium to a final concentration of 250 µg/ml; a conditioned medium, which was the supernatant obtained by inoculating CHO-K1 in the subculture medium, culturing for 1 day, and then performing sterile filtration; a cloning medium, which was prepared by adding 1% GlutaMAX to 75%(v/v) EX-CELL CHO Cloning Medium, 20%(v/v) conditioned medium, and 5%(v/v) ClonaCell-CHO ACF Supplement; and in fed-batch medium, basal medium was prepared by adding 1% GlutaMAX (ThermoFisher) to EX-CELL Advanced CHO Fed-batch Medium, and the feed medium was Cell Boost 7a/7b (HyClone).

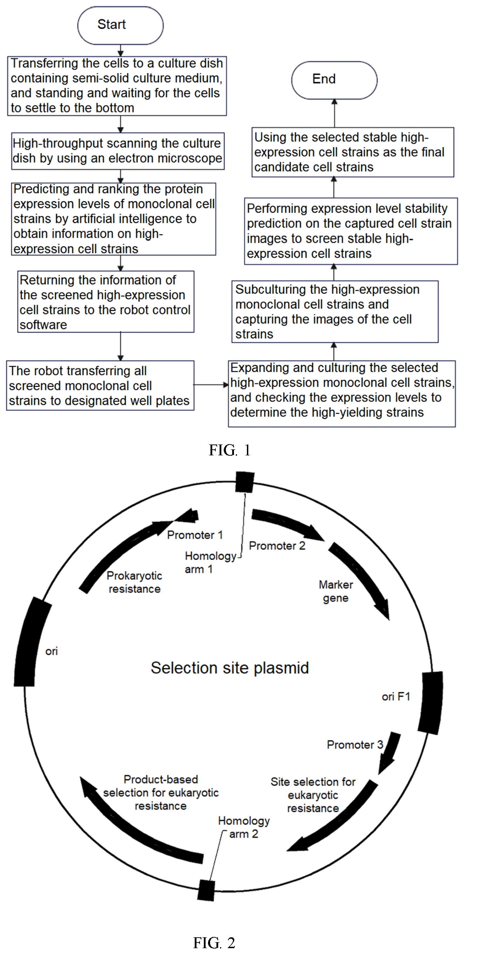

The operation flow of the present example was as shown in FIG. 1, and mainly includes the following steps:
(1) using the green fluorescent protein gene (EGFP) as the screening marker gene and using the attP sequence as the homology arm to construct a recombinant plasmid containing RMCE (recombinase-mediated cassette exchange), as shown in FIG. 2; linearizing the constructed plasmid, and then purifying and recovering the linear DNA; collecting CHO-K1 cells, centrifuging, and discarding the supernatant; resuspending the cells by using 100 µL of resuspension buffer R Buffer dedicated for cell electroporation instrument; and performing electroporation for 3 times.
(2) applying selective pressure with a selective pressure reagent of G418 at a selective pressure concentration of 800 µg/ml, dividing into minipools (i.e., 96-well plate) and culturing;
(3) checking the plates after ten days, performing enrichment when a large number of fluorescent cell clusters were observed, where the principle of one well to one well was followed during the enrichment;
(4) observing the growth status of the enriched cells at any time, and expanding after the coverage rate reached 50% or above;
(5) after expanding to shake flasks, subculturing for three times to form a stable cell pool, where the formation of monoclonal cells and the screening of stable fluorescence cells were achieved by artificial intelligence, the steps were as shown in FIG. 1, and the specific steps were as shown in a) -h):
   a) diluting the cells in the stable cell pool to a certain concentration, and then inoculating into culture dishes of semi-solid culture medium, where during the inoculation process, the cells were ensured to be distributed approximately evenly in various positions of the culture dishes; and standing for about half an hour to allow the cells to settle to the bottom of the culture dishes;
   b) transferring each of the culture dishes to the stage of the electron microscope, and performing high-throughput scanning on the cells in the culture dish by using the electron microscope;
   c) uploading the image scanned by the electron microscope to the server for artificial intelligence image analysis, where the analysis process included detection of monoclonal cell strains, prediction of protein expression levels of monoclonal cell strains, ranking of the protein expression levels, and encoding and localization of the screened high-expressing protein cell strains; and the prediction of protein expression levels of monoclonal cell strains may be fluorescence-based or non-fluorescence-based, and the following step c was the non-fluorescence-based prediction of protein expression levels;
      where step c involved object detection of monoclonal cell strain based on image processing technique; in the present example, the YOLOv8 object detection algorithm was used, and the actual detection performance achieved a mean Average Precision (mAP) of 94.2%; the object detection model for monoclonal cell strains in step c was consistent with commonly used deep learning object detection models, so it was not described in detail; specifically, the cell images need to be labeled first; to improve the prediction accuracy of the algorithm, the bounding boxes of all monoclonal cell strains and adherent cell strains were labeled as ground truth object bounding boxes for loss calculation of the model output; through algorithm training, the model learned the ability to extract the bounding box information of monoclonal cell strains; and in step c of the practical application scenario, the trained model may predict the bounding boxes of monoclonal cell strains in the images;
      step c involved the prediction of expression levels of fluorescent proteins based on monoclonal cell images; in the present example, the SqueezeNet deep learning network and MSE Loss loss function were used to predict and rank protein expression levels in this project, and the ranking result was the final goal of the algorithm, the Normalized Discounted Cumulative Gain (NDCG) was used as the evaluation criterion for ranking algorithm herein; and the specific calculation formula was as follows: $DCG = {\sum }_{i = 1}^{k} \frac{rel \left(i\right)}{{log}_{2} \left(i+1\right)}$ $NDCG = \frac{DCG}{IDCG}$
      where IDCG=the best-ranked DCG; specifically, in the present example, the expression levels were predicted from cell images, and the ranking based on the predicted expression levels was compared with the ranking results of the actual fluorescence values, with NDCG=0.89; and the expression level prediction model in the present application was described in detail in patent CN112037862B;
   d) returning the coding and localization information of the screened cell strains with high protein expression to the robot control software;
      where for the cell codes and positions returned in step d), in the present example, the top 100 cells in terms of the predicted expression levels were returned, with the codes assigned sequentially from 1 to 100; and the position information including coordinates in a planar coordinate system relative to the center position of the microscope (culture medium depth was not considered temporarily, because all cells to be selected settled to the bottom of the culture dish; and in addition, during the imaging process, cells that did not settle to the bottom of the culture dish may be out of focus, resulting in blurred cell images, and the cells with blurred images may also be excluded);
   (e) using robot control software to automatically operate (or manually assist) the robotic arm to aspirate the screened monoclonal cell strains and transfer into a designated well plate, and repeating this process until all high-expression cell strains were transferred;
      where the process of aspirating the target monoclonal cell strains and transferring to the well plate by the robotic arm as described in step e was described in detail in patents CN113821287B, CN113403431B, CN113771030A, and CN113733087B;
   f) cloning culturing the cells in the well plate to a certain number, then transferring to larger well plate for culturing, finally expanding to shake flask culture, and detecting the cell expression level to determine whether the cell strains selected by artificial intelligence were high-yielding cell strains;
      where in the present example, step f involved transferring to a 96-well plate for scale-up culture, and then transferring to a shake flask for culturing;
   g) subculturing the selected high-yielding cell strains, where before the subculturing of each generation of cell strains, a part of the samples were diluted, placed in a culture dish, and imaged with an electron microscope, and images of the cell strains are continuously captured for several generations; and currently, the expression condition of selected cells may be predicted from captured images by using a model after cell morphology learning, and the expression condition of selected cells may also be determined by using fluorescent labeling;
      where for the imaging of the monoclonal cells in step g in the present example, a Thermo Fisher Scientific M7000 electron microscope was used for the imaging.
   h) inputting the collected images of cell strains of several generations into the artificial intelligence algorithm, predicting the subculture stability of the cell strains according to fluorescence intensities and/or protein expression levels, and selecting cell strains with high protein expression characteristics and stable subculturing as the final candidate cell strains, where the subculture stability prediction of the cell strain may be based on fluorescence or not on fluorescence but on cell morphology, and the following step h was the prediction of protein expression level based on cell morphology instead of fluorescence; and
      where step h predicted the subculture stability of cell strains, including but not limited to using traditional image recognition algorithms to extract image features by using histograms and perform prediction, using deep learning neural network to automatically extract image features and perform prediction, and predicting by other image-based techniques; and in the present example, a deep learning-based method was used to automatically extract image features of stable cell strains and unstable cell strains and predict the stability, achieving a prediction result accuracy of 84% for different cell strains, which was described in detail in patent CN114417582A;
      In the above, step d and step e may be completed manually; and in the present example, they were completed automatically by a robotic arm;
(6) screening out and eliminating monoclonal clones with a growth rate lower than CHO-K1 by counting on the day of inoculation, the 3rd day of culture, the 5th day of culture, the 7th day of culture, the 9th day of culture, the 11th day of culture, the 13th day of culture, and the 14th day of culture, where the CHO-K1 cell was used as a control, the inoculation density was 5×10⁵ cells/ml, and the inoculation system was 30ml; and
(7) resuscitating the remaining monoclonal clones and performing stable subculture after recovery, where the subculture was performed every 3 or 4 days, with the cell density of 3×10⁵ cells/ml for the 4-day subculture, and the cell density of 5×10⁵ cells/ml for the 3-day subculture, and simultaneously monitoring the fluorescence value of the cell strains regularly during the subculture, performing a subculture stability study for approximately 90 days on the aforementioned high-fluorescence cell strains by using the subculture medium and identifying GBB003 cells with small fluorescence variation and stable cell strain growth as stable high-fluorescence cells; performing Next-Generation Sequencing (NGS) on the integration sites of the cell strains, where the determined sequence of the high-expression fragment at the integration site was SEQ ID No: 1, and specifically, the annotation information of the integration site in CHO was: NW_003616785.1:83044.

### Example 2: Construction of Cell Strain

### (1) Construction of Exogenous Nucleic Acid

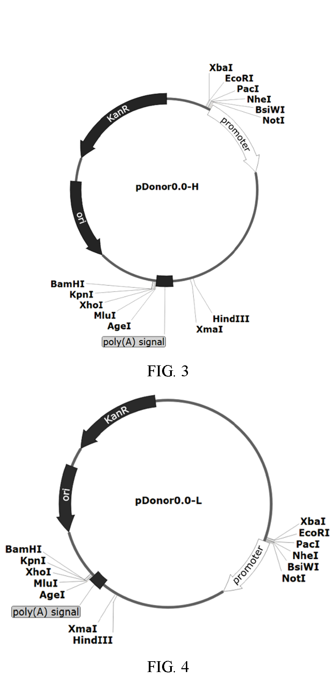

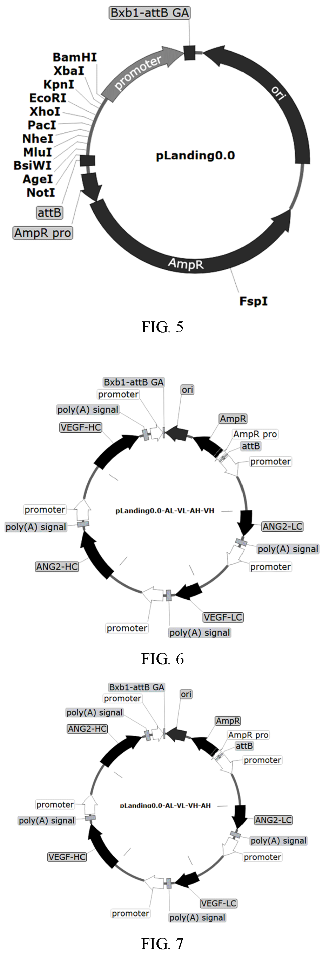

The present application provided an approach for constructing a multi-reading frame plasmid, as shown in FIG. 3. In the above, pDonor0.0-H was used to construct a vector for an expression reading frame of the heavy chain, and the target gene sequence may be inserted between the XmaI and/or HindIII restriction sites, where KanR provided a kanamycin resistance gene used for the screening during plasmid construction; the Ori region was the replication initiation site of the plasmid, used for plasmid replication and amplification in prokaryotic cells; the CMV region (not shown in the figure) was used to initiate the expression of the target gene; SV40 poly(A) was used to terminate the transcription of the target gene; and NotI and other restriction sites were used for inserting a complete reading frame into the pLanding0.0 plasmid via restriction digestion and ligation. As shown in FIG. 4, pDonor0.0-L was used to construct a vector for an expression reading frame for the light chain, where KanR provided a kanamycin resistance gene used for the screening during plasmid construction; the Ori region was the replication initiation site of the plasmid, used for plasmid transcription and amplification in prokaryotic cells; the CMV region (not shown in the figure) was used to initiate the expression of the target gene; SV40 poly(A) was used to terminate the transcription of the target gene; XmaI and/or HindIII restriction sites were used for inserting the target gene sequence; and NotI and other restriction sites were used for inserting the complete reading frame into the pLanding0.0 plasmid via restriction digestion and ligation. The pLanding0.0 was used to accept reading frames from pDonor0.0-H and/or pDonor0.0-L. The pLanding0.0 vector is as shown in FIG. 5, where the Ori region was the replication initiation site of the plasmid, used for plasmid transcription and amplification in prokaryotic cells; and AmpR provided an ampicillin resistance gene used for the screening during plasmid construction.

In the present example, the target gene arrangements within the constructed plasmid structure were designated as Molecule 1, Molecule 2, Molecule 3, and Molecule 4, respectively.

The designed construction method of Molecule 1 involved synthesizing and inserting the kozak sequence, signal peptide sequence (SEQ ID No: 6), and ANG-2 antibody light chain sequence (coding sequence: SEQ ID No: 2) between the HindIII and Xma I restriction sites of the pDonor0.0-L plasmid to form a pDonor0.0-ANG-2-LC plasmid; synthesizing and inserting the kozak sequence, signal peptide sequence (SEQ ID No: 6), and VEGF antibody light chain sequence (coding sequence: SEQ ID No: 4) between the HindIII and XmaI restriction sites of the pDonor0.0-L plasmid to form a pDonor0.0-VEGF-2-LC plasmid; synthesizing and inserting the kozak sequence, signal peptide sequence (SEQ ID No: 7), and ANG-2 antibody heavy chain sequence (coding sequence: SEQ ID No: 3) between the HindIII and XmaI restriction sites of the pDonor0.0-H plasmid to form a pDonor0.0-ANG-2-HC plasmid; synthesizing and inserting the kozak sequence, signal peptide sequence (SEQ ID No: 7), and VEGF antibody heavy chain sequence (coding sequence: SEQ ID No. 5) between the HindIII and XmaI restriction sites of the pDonor0.0-H plasmid to form a pDonor0.0-VEGF-2-HC plasmid; double-digesting the pDonor0.0-ANG-2-LC and pLanding0.0 plasmids with NotI/AgeI respectively, then recovering the ANG-2-LC reading frame fragment from pDonor0.0-ANG-2-LC and recovering the linearized pLanding0.0 fragment, then ligating by using T4 DNA ligase and transforming into DH5α chemically competent cells, spreading on LB ampicillin-resistant plates, and after colonies grew, rinsing and collecting bacterial cells by using LB medium, extracting the plasmid to form pLanding0.0-AL; double-digesting the pDonor0.0-VEGF-LC and pLanding0.0-AL plasmids with Bsiwl/MluI respectively, then recovering the VEGF-LC reading frame fragment from pDonor0.0-VEGF-LC and recovering the linearized pLanding0.0-AL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL; double-digesting the pDonor0.0-ANG-2-HC and pLanding0.0-AL-VL plasmids with NheI/XhoI respectively, then recovering the ANG-2-HC reading frame fragment from pDonor0.0-ANG-2-HC and recovering the linearized pLanding0.0-AL-VL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL-AH; double-digesting the pDonor0.0-VEGF-HC and pLanding0.0-AL-VL-AH plasmids with EcoRI/KpnI respectively, then recovering the VEGF-HC reading frame fragment from pDonor0.0-VEGF-HC and recovering the linearized pLanding0.0-AL-VL-AH fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL-AH-VH. In the present example, the ANG-2 antibody light chain sequence is the first target gene, the VEGF antibody light chain sequence is the second target gene, the ANG-2 antibody heavy chain sequence is the third target gene, and the VEGF antibody heavy chain sequence is the fourth target gene, and the schematic view of the plasmid is as shown in FIG. 6.

The designed construction of Molecule 2 involved double-digesting the pDonor0.0-ANG-2-LC and pLanding0.0 plasmids with NotI/AgeI respectively, then recovering the ANG-2-LC reading frame fragment from pDonor0.0-ANG-2-LC and recovering the linearized pLanding0.0 fragment, then ligating by using T4 DNA ligase and transforming into DH5α chemically competent cells, spreading on LB ampicillin-resistant plates, and after colonies grew, rinsing and collecting bacterial cells by using LB medium, and extracting the plasmid to form pLanding0.0-AL; double-digesting the pDonor0.0-VEGF-LC and pLanding0.0-AL plasmids with Bsiwl/MluI respectively, then recovering the VEGF-LC reading frame fragment from pDonor0.0-VEGF-LC and recovering the linearized pLanding0.0-AL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL; double-digesting the pDonor0.0-VEGF-HC and pLanding0.0-AL-VL plasmids with Nhel/Xhol respectively, then recovering the VEGF-HC reading frame fragment from pDonor0.0-VEGF-HC and recovering the linearized pLanding0.0-AL-VL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL-VH; double-digesting the pDonor0.0-ANG-2-HC and pLanding0.0-AL-VL-AH plasmids with EcoRI/KpnI respectively, then recovering the ANG-2-HC reading frame fragment from pDonor0.0-ANG-2-HC and recovering the linearized pLanding0.0-AL-VL-AH fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-AL-VL-VH-AH. In the present example, the ANG-2 antibody light chain sequence is the first target gene, the VEGF antibody light chain sequence is the second target gene, the VEGF antibody heavy chain sequence is the third target gene, and the ANG-2 antibody heavy chain sequence is the fourth target gene, and the schematic view of the plasmid is as shown in FIG. 7.

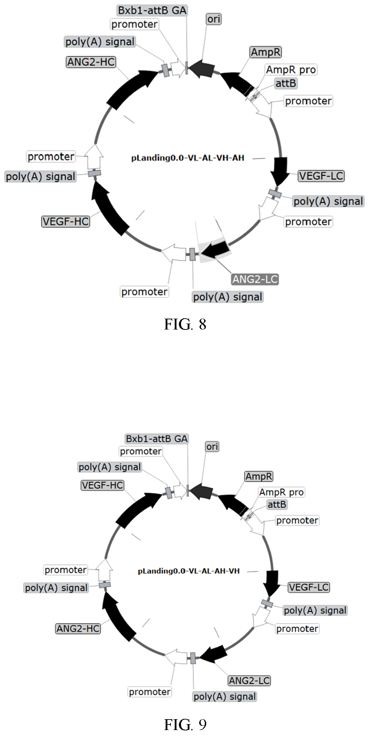

The designed construction of Molecule 3 involved double-digesting the pDonor0.0-VEGF-LC and pLanding0.0 plasmids with NotI/AgeI respectively, then recovering the VEGF-LC reading frame fragment from pDonor0.0-VEGF-LC and recovering the linearized pLanding0.0 fragment, then ligating by using T4 DNA ligase and transforming into DH5α chemically competent cells, spreading on LB ampicillin-resistant plates, and after colonies grew, rinsing and collecting bacterial cells by using LB medium, and extracting the plasmid to form pLanding0.0-VL; double-digesting the pDonor0.0-ANG-2-LC and pLanding0.0-VL plasmids with Bsiwl/MluI respectively, then recovering the ANG-2-LC reading frame fragment from pDonor0.0-ANG-2-LC and recovering the linearized pLanding0.0-VL fragment, then ligating by using T4 DNA ligase and transforming into Stbl3 chemically competent cells, spreading on LB ampicillin-resistant plates, and after colonies grew, rinsing and collecting bacterial cells by using LB medium, and extracting the plasmid to form pLanding0.0-VL-AL; double-digesting the pDonor0.0-VEGF-HC and pLanding0.0-VL-AL plasmids with Nhel/Xhol respectively, then recovering the VEGF-HC reading frame fragment from pDonor0.0-VEGF-HC and recovering the linearized pLanding0.0-VL-AL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-VL-AL-VH; double-digesting the pDonor0.0-ANG-2-HC and pLanding0.0-VL-AL-VH plasmids with EcoRI/KpnI respectively, then recovering the ANG-2-HC reading frame fragment from pDonor0.0-ANG-2-HC and recovering the linearized pLanding0.0-VL-AL-VH fragment, then ligating by using T4 DNA ligase and transforming into Stbl3 chemically competent cells, spreading on LB ampicillin-resistant plates, and after colonies grew, rinsing and collecting bacterial cells by using LB medium, and extracting the plasmid to form pLanding0.0-VL-AL-VH-AH. In the present example, the VEGF antibody light chain sequence is the first target gene, the ANG-2 antibody light chain sequence is the second target gene, the VEGF antibody heavy chain sequence is the third target gene, and the ANG-2 antibody heavy chain sequence is the fourth target gene, and the schematic view of the plasmid is as shown in FIG. 8.

The designed construction of Molecule 4 involved double-digesting the pDonor0.0-VEGF-LC and pLanding0.0 plasmids with NotI/AgeI respectively, then recovering the VEGF-LC reading frame fragment from pDonor0.0-VEGF-LC and recovering the linearized pLanding0.0 fragment, then ligating by using T4 DNA ligase and transforming into DH5α chemically competent cells, spreading on LB ampicillin-resistant plates, and after colonies grew, rinsing and collecting bacterial cells by using LB medium, and extracting the plasmid to form pLanding0.0-VL; double-digesting the pDonor0.0-ANG-2-LC and pLanding0.0-VL plasmids with Bsiwl/MluI respectively, then recovering the ANG-2-LC reading frame fragment from pDonor0.0-ANG-2-LC and recovering the linearized pLanding0.0-VL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-VL-AL; double-digesting the pDonor0.0-ANG-2-HC and pLanding0.0-VL-AL plasmids with Nhel/Xhol respectively, then recovering the ANG-2-HC reading frame fragment from pDonor0.0-ANG-2-HC and recovering the linearized pLanding0.0-VL-AL fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-VL-AL-AH; double-digesting the pDonor0.0-VEGF-HC and pLanding0.0-VL-AL-AH plasmids with EcoRI/KpnI respectively, then recovering the VEGF-HC reading frame fragment from pDonor0.0-VEGF-HC and recovering the linearized pLanding0.0-VL-AL-AH fragment, then ligating by using T4 DNA ligase and transforming into StBl3 chemically competent cells, spreading on LB ampicillin-resistant plates, and after colonies grew, rinsing and collecting bacterial cells by using LB medium, and extracting the plasmid to form pLanding0.0-VL-AL-AH-VH. In the present example, the VEGF antibody light chain sequence is the first target gene, the ANG-2 antibody light chain sequence is the second target gene, the ANG-2 antibody heavy chain sequence is the third target gene, and the VEGF antibody heavy chain sequence is the fourth target gene, and the schematic view of the plasmid is as shown in FIG. 9.

The present example was used to express an ANG-2/VEGF bispecific bivalent antibody, where the antibody contained the heavy chain sequence (coding sequence SEQ ID No: 3) and light chain sequence (coding sequence SEQ ID No: 2) corresponding to the ANG-2 target, and the heavy chain sequence (coding sequence SEQ ID No: 5) and light chain sequence (coding sequence SEQ ID No: 4) corresponding to the VEGF target, all genes were synthesized by GenScript, and a part of the information was as follows.

In the ANG-2 antibody light chain sequence, the coding sequence of the heavy chain constant region 1 (CH1) in the first light chain gene was SEQ ID No: 8.

In the ANG-2 antibody heavy chain sequence, the coding sequence of the light chain constant region (CL) in the first heavy chain gene was SEQ ID No: 9.

In the above, a signal peptide sequence SEQ ID No: 6 required for antibody secretion was provided before the antibody light chain sequence.

In the above, a signal peptide sequence SEQ ID No: 7 required for antibody secretion was provided before the antibody heavy chain sequence.

| No. | Name | Arrangement Order of Target Genes in Exogenous Nucleic Acids |
|---|---|---|
| 1 | Molecule 1 | (ANG-2 LC)-(VEGF LC)-(ANG-2 HC)-(VEGF HC) |
| 2 | Molecule 2 | (ANG-2 LC)-(VEGF LC)-(VEGF HC)-(ANG-2 HC) |
| 3 | Molecule 3 | (VEGF LC)-(ANG-2 LC)-(VEGF HC)-(ANG-2 HC) |
| 4 | Molecule 4 | (VEGF LC)-(ANG-2 LC)-(ANG-2 HC)-(VEGF HC) |

In the ANG-2/VEGF bispecific bivalent antibody formed after the expression of four molecules, the constant region CL of the ANG-2 antibody light chain sequence and the constant region CH1 within the ANG-2 antibody heavy chain sequence were mutually exchanged. Therefore, the expression difficulty of the ANG-2 antibody light chain sequence was higher than that of the VEGF antibody light chain sequence, and the expression difficulty of the ANG-2 antibody heavy chain sequence was higher than that of the VEGF antibody heavy chain sequence.

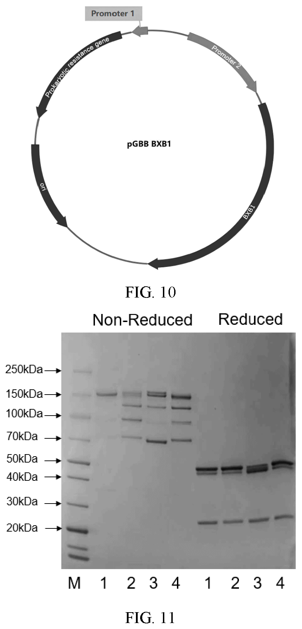

(2) Selecting stable high-fluorescence cells GBB003 for transfection, integration and verification, amplifying and linearizing the recombinant plasmids of the ANG-2/VEGF bispecific bivalent antibody expression genes in the four arrangement manners included in aforementioned step (1), then co-transfecting into stable high-fluorescence cells GBB003 with Bxb-1 integrase respectively, where the Bxb-1 integrase plasmid map is as shown in FIG. 10.

(3) One day after the transfection, constructing a minipool from the cell pool and performing selective pressure screening by using the selective pressure medium 2.

(4) After 14 days, expanding and culturing the non-fluorescent cells in the minipool, where the manner of the expanding was 96-well to 24-well plate to 6-well plate, in the above, when expanding to 6-well plates, 6-well batch culture was performed, the 6-well batch culture was performed by inoculating at 5×10⁵ cells/mL in a 2 mL system in 6-well plates, and culturing under conditions of 37 °C, 5% CO₂, and 120 rpm. The experimental results of the 6-well batch culture are as shown in Table 1.

**Table 1: Statistical Results of Expression Levels in 6-well Batch Culture of Cell Pool**

| Molecule No. | Cell Pool No. | Expression level in 6-well Batch Culture (mg/L) | Molecule No. | Cell Pool No. | Expression level in 6-well Batch Culture (mg/L) |
|---|---|---|---|---|---|
| Molecule 1 | 1-3 | 634.5 | Molecule 2 | 2-1 | 482.9 |
| | 1-6 | 959.6 | | 2-2 | 472.6 |
| | 1-9 | 1026.2 | | 2-5 | 525.0 |
| | 1-10 | 780.6 | | 2-6 | 472.3 |
| | 1-11 | 799.9 | | 2-9 | 462.1 |
| Molecular 3 | 3-1 | 601.4 | Molecule 4 | 4-1 | 881.9 |
| | 3-2 | 536.3 | | 4-3 | 755.2 |
| | 3-3 | 613.0 | | 4-4 | 851.9 |
| | 3-4 | 704.2 | | 4-6 | 1203.0 |
| | 3-9 | 491.0 | | 4-9 | 865.4 |

(5) Selecting the top 1 cell pool for each molecule (i.e., cell pool 1-9, cell pool 2-5, cell pool 3-4, and cell pool 4-6), expanding to T125 shake flasks, subculturing twice, then performing shake flask feeding (i.e., fed-batch culture in shake flasks) for 14 days, where the inoculating density was 5×10⁵/ml, and the volume of the inoculating system was 30 ml; counting on the day of inoculation, the 3rd day of culture, the 5th day of culture, the 7th day of culture, the 9th day of culture, the 11th day of culture, the 13th day of culture, and the 14th day of culture, supplementing 3% Cell Boost 7a and 0.3% Cell Boost 7b on the 3rd day, supplementing 5% Cell Boost 7a and 0.5% Cell Boost 7b on the 5th day of culture, the 7th day of culture, the 9th day of culture, the 11th day of culture, and the 13th day of culture, where during this period, the glucose concentration was controlled at the level of 2-8 g/L, and the expression level was measured by using Octect after 14 days. The expression level results of shake flask feeding are as shown in Table 2.

**Table 2: Statistical Results of Expression levels of Shake Flask Feeding**

| Molecule No. | Cell pool No. | Shake flask Expression (mg/L) | Molecule No. | Cell pool No. | Shake flask Expression (mg/L) |
|---|---|---|---|---|---|
| Molecule 1 | 1-9 | 3915.6 | Molecule 2 | 2-5 | 3496.4 |
| Molecule 3 | 3-4 | 4077.4 | Molecule 4 | 4-6 | 2321.1 |

(6) Purifying the supernatant from the shake flask feed (i.e., shake flask fed-batch culture) by using a Protein A chromatography column, and then performing SDS-PAGE analysis (results as shown in FIG. 11) and SEC purity analysis, with the SEC purity as shown in Table 3, where the SEC purity of Molecule 1 was 83.47%, significantly better than that of other molecular designs; analyzing the proportion of each chain by reduced CE-SDS analysis, as shown in Table 4, where the ratio of the two light chains and two heavy chains in Molecule 1 is closer to 1:1, better meeting the expected theoretical value. Since based on comprehensive analysis, Molecule 1 showed the best purity and molecule expression level, cell pool 1-9 of Molecule 1 was selected for monoclonal cloning, while cell pools 2-5, 3-4, and 4-6 served as control groups for monoclonal cloning.

**Table 3: Results of SEC Purity Analysis of Cell Pools**

| Molecule No. | Cell Pool No. | SEC Purity (%) | Molecule No. | Cell Pool No. | SEC Purity (%) |
|---|---|---|---|---|---|
| Molecule 1 | 1-9 | 83.47 | Molecule 2 | 2-5 | 53.12 |
| Molecule 3 | 3-4 | 70.12 | Molecule 4 | 4-6 | 51.38 |

**Table 4: Results of Reduced CE-SDS Purity Analysis for Cell Pools**

| Molecule No. | Cell Pool No. | L1 (%) | L2 (%) | H1 (%) | H2 (%) |
|---|---|---|---|---|---|
| Molecule 1 | 1-9 | 15.1 | 14 | 30.9 | 40 |
| Molecule 2 | 2-5 | 0 | 23.9 | 22.8 | 53.3 |
| Molecule 3 | 3-4 | 21.7 | 7.7 | 31.8 | 38.8 |
| Molecule 4 | 4-6 | 15.6 | 12 | 27.2 | 45.1 |

(7) Performing limited dilution on cell pools 1-9, 2-5, 3-4, and 4-6, plating a 96-well plate according to a density of 0.8 Cell/well, imaging cell growth status at 1st h, 1st day, 2nd day, 3rd day, 5th day, 7th day, and 14th day by verified in situ plate seeding (VIPS), and counting non-fluorescent monoclonal colonies.

(8) Expanding the non-fluorescent monoclonal wells, and by following the manner of 96-well plate to 24-well plate to 6-well plate to shake flask, eliminating clones with low growth rates during the expansion process, where for 4-6, no monoclonal clones were formed and all the cells did not grow or proliferate, therefore, the cell pool 4-6 could not be used for 6-well batch culture screening, where 6-well batch culture was performed after expansion in 6-well, the 6-well batch culture was performed by inoculating at 5×10⁵ /mL in a 2 mL system in 6-well plates, culturing under conditions of 37°C, 5% CO₂, and120 rpm for 7 days. The top 5 monoclonal clones with the highest expression levels in the 6-well batch culture of each cell pool are as shown in Table 5.

**Table 5: Statistical Results of Expression levels in Monoclonal Clone 6-well Batch Cultures**

| Cell Pool | Monoclonal Clone No. | Expression level in 6-Well Batch Culture (mg/L) |
|---|---|---|
| 1-9 | 1-9-1 | 1485.7 |
| | 1-9-2 | 1408.2 |
| | 1-9-3 | 1193.3 |
| | 1-9-4 | 653.4 |
| | 1-9-5 | 649.5 |
| 2-5 | 2-5-1 | 1215.9 |
| | 2-5-2 | 686.1 |
| | 2-5-3 | 683.9 |
| | 2-5-4 | 679.1 |
| | 2-5-5 | 657.6 |
| 3-4 | 3-4-1 | 676.0 |
| | 3-4-2 | 633.2 |
| | 3-4-3 | 601.2 |
| | 3-4-4 | 598.6 |
| | 3-4-5 | 581.9 |

(9) Selecting the top three cell strains with the highest expression levels in 6-well batch culture for each cell pool, subculturing twice, and then performing shake flask feeding for 14 days, where the inoculating density was 5×10⁵/ml, and the volume of the inoculating system was 30 ml; counting on the day of inoculation, the 3rd day of culture, the 5th day of culture, the 7th day of culture, the 9th day of culture, the 11th day of culture, the 13th day of culture, and the 14th day of culture, supplementing 3% Cell Boost 7a and 0.3% Cell Boost 7b on the 3rd day, supplementing 5% Cell Boost 7a and 0.5% Cell Boost 7b on the 5th day of culture, the 7th day of culture, the 9th day of culture, the 11th day of culture, and the 13th day of culture, where during this period, the glucose concentration was controlled at the level of 2-8 g/L, and the expression level was measured by using Octect^{®} after 14 days. The expression level results of the monoclonal shake flask feeding are as shown in Table 6.

**Table 6: Statistical Results of Expression levels in Monoclonal Shake Flask Feeding**

| Cell Pool | Monoclonal Clone No. | Expression levels in Shake Flask Feeding (mg/L) |
|---|---|---|
| 1-9 | 1-9-1 | 5946.7 |
| | 1-9-2 | 5289.1 |
| | 1-9-3 | 3849.0 |
| 2-5 | 2-5-1 | 4786.7 |
| | 2-5-2 | 4215.5 |
| | 2-5-3 | 3801.2 |
| 3-4 | 3-4-1 | 4384.4 |
| | 3-4-2 | 4282.2 |
| | 3-4-3 | 3765.4 |

The supernatant from the shake flask feed was captured by using Protein A packing material and then subjected to an SEC analysis. The results are as shown in Table 7. The quality attributes of the monoclonal cells produced by each cell pool are relatively uniform, where the cell pool of Molecule 1 shows the highest SEC purity. Through calculating the yield of the target molecule, both 1-9-1 and 1-9-2 exhibit excellent performance, where the theoretical yield of the target molecule is approximately 4.7 g. The calculation results are as shown in Table 8.

**Table 7: Results of Monoclonal SEC Analysis**

| Cell Pool | Monoclonal Clone No. | SEC Purity (%) |
|---|---|---|
| 1-9 | 1-9-1 | 80.60 |
| | 1-9-2 | 89.77 |
| | 1-9-3 | 88.41 |
| 2-5 | 2-5-1 | 67.35 |
| | 2-5-2 | 67.55 |
| | 2-5-3 | 67.59 |
| 3-4 | 3-4-1 | 75.64 |
| | 3-4-2 | 76.01 |
| | 3-4-3 | 73.27 |

**Table 8: Relative Yield Statistic Table of Monoclonal Target Products**

| Cell Pool | Monoclonal Clone No. | Relative Yield of Target Product |
|---|---|---|
| 1-9 | 1-9-1 | 4793.0 |
| | 1-9-2 | 4748.0 |
| | 1-9-3 | 3402.9 |
| 2-5 | 2-5-1 | 3223.8 |
| | 2-5-2 | 2847.6 |
| | 2-5-3 | 2569.2 |
| | 3-4-1 | 3316.4 |
| 3-4 | 3-4-2 | 3254.9 |
| | 3-4-3 | 2758.9 |

Through the above experiment contents, we screen the expected bispecific antibody expressing cell strains, where the experimental procedure is controllable, workload is significantly reduced compared with traditional methods, and the antibody mismatch rate is lower. In the ANG-2/VEGF bispecific bivalent antibodies formed after expressing the four molecules, the constant region CL of the ANG-2 antibody light chain sequence and the constant region CH1 within the ANG-2 antibody heavy chain sequence are mutually exchanged. Therefore, the expression difficulty of the ANG-2 antibody light chain sequence is higher than that of the VEGF antibody light chain sequence, and the expression difficulty of the ANG-2 antibody heavy chain sequence is higher than that of the VEGF antibody heavy chain sequence. Furthermore, since artificial sequence exchange is more likely to cause mismatches, the expression reading frame of ANG-2 is placed at the front of the plasmid design so that it is less affected by the preceding reading frame. In conventional antibody expression, the expression of the heavy chain depends on the correct folding of the light chain, and the light chain may assist in the secretion of the heavy chain. Therefore, that the reading frame of the light chain is placed at the front of the plasmid design enables the generation of more light chain antibody fragments, thereby further improving the yield of the target molecule. Therefore, this explains the reason of our Molecule 1 design achieving higher expression level and lower mismatch rate.

The exogenous nucleic acid fragment may be replaced. The first target gene may be replaced with a gene encoding the first protein, the second target gene may be replaced with a gene encoding the second fusion protein, and the expression difficulty of the first protein is higher than that of the second protein. The first target gene may be replaced with a gene encoding the monoclonal antibody light chain, the second target gene may be replaced with a gene encoding the monoclonal antibody heavy chain, and the expression difficulty of the light chain is higher than that of the heavy chain. The first target gene may be replaced with a gene encoding the monoclonal antibody heavy chain, the second target gene may be replaced with a gene encoding the monoclonal antibody light chain, and the expression difficulty of the heavy chain is higher than that of the light chain. An exogenous nucleic acid fragment may contain multiple target genes, where the target genes differ in the difficulty of protein expression, and the target gene with higher expression difficulty is positioned before the target gene with lower expression difficulty within the exogenous nucleic acid fragment.

It should be noted that the above are merely preferred embodiments and the technical principles employed in the present application. Those skilled in the art may understand that the present application is not limited to the specific embodiments described herein, and various obvious changes, readjustments, and substitutions may be made by those skilled in the art without departing from the scope of protection of the present application. Therefore, although the present application is described in detail through the above embodiments, the present application is not limited to the above embodiments. Many other equivalent embodiments may be included without departing from the technical concept of the present application, all of which fall within the scope of protection of the present application.

Those skilled in the art may also recognize, or be able to identify, by using no more than conventional experiments, many equivalents to the specific embodiments of the present application described herein. These equivalents are intended to be included in the appended claims.

## Claims

1. A cell strain, wherein the cell strain comprises a high-expression fragment and an exogenous nucleic acid fragment integrated within the high-expression fragment;
the high-expression fragment contains a nucleotide sequence as shown in SEQ ID No: 1; and
the exogenous nucleic acid fragment comprises a first target gene encoding a first protein fragment and a second target gene encoding a second protein fragment, an expression difficulty of the first protein fragment is higher than that of the second protein fragment, and the first target gene is located upstream of the second target gene.

2. The cell strain according to claim 1, wherein the exogenous nucleic acid fragment further comprises a third target gene encoding a third protein fragment and a fourth target gene encoding a fourth protein fragment, wherein an expression difficulty of the third protein fragment is higher than that of the fourth protein fragment, the third target gene is located upstream of the fourth target gene, wherein the first target gene, the second target gene, the third target gene and the fourth target gene jointly encode a bispecific antibody or an antigen-binding fragment thereof.

3. The cell strain according to claim 2, wherein the second target gene is located upstream of the third target gene.

4. The cell strain according to claim 3, wherein the first protein fragment is a first light chain, the second protein fragment is a second light chain, the third protein fragment is a first heavy chain, and the fourth protein fragment is a second heavy chain; optionally, a protein formed by binding of the first light chain and the first heavy chain can specifically bind to a first target, and the protein formed by binding of the second light chain and the second heavy chain can specifically bind to a second target.

5. The cell strain according to claim 4, wherein a sequence of the first target gene is as shown in SEQ ID No: 2, a sequence of the third target gene is as shown in SEQ ID No: 3, a sequence of the second target gene is as shown in SEQ ID No: 4, and a sequence of the fourth target gene is as shown in SEQ ID No: 5.

6. The cell strain according to claim 5, wherein the first target gene and the second target gene each comprise upstream a nucleotide sequence encoding a first signal peptide as shown in SEQ ID No: 6, and the third target gene and the fourth target gene each comprise upstream a nucleotide sequence encoding a second signal peptide as shown in SEQ ID No: 7.

7. The cell strain according to any one of claims 1 to 6, wherein an integration site of the exogenous nucleic acid fragment is any site within a base interval from positions 11 to 430 of the high-expression fragment; and
the integration site of the exogenous nucleic acid fragment is a position indicated by an annotation information NW_003616785.1:83044 in a CHO cell.

8. The cell strain according to claim 7, wherein the cell strain is the CHO cell.

9. A preparation method for the cell strain according to any one of claims 1 to 8, wherein the preparation method comprises following steps: site-specifically integrating the exogenous nucleic acid fragment into the high-expression fragment to obtain the cell strain.

10. The preparation method according to claim 9, wherein the preparation method comprises following steps:
S1: providing the exogenous nucleic acid fragment, wherein the exogenous nucleic acid fragment comprises, in a 5'-3' direction, a first target gene encoding a first light chain, a second target gene encoding a second light chain, a third target gene encoding a first heavy chain, and a fourth target gene encoding a second heavy chain in sequence, wherein an expression difficulty of the first target gene is higher than that of the second target gene, and an expression difficulty of the third target gene is higher than that of the fourth target gene; and the first target gene, the second target gene, the third target gene, and the fourth target gene are each independently provided upstream with a nucleotide sequence encoding a signal peptide, and
the first target gene, the second target gene, the third target gene, and the fourth target gene jointly encode the bispecific antibody or the antigen-binding fragment thereof; and
S2. site-specifically integrating the exogenous nucleic acid fragment into the high-expression fragment to obtain the cell strain.

11. Use of the cell strain according to any one of claims 1 to 8 in protein expression.
